Europäisches Patentamt

(19) European Patent Office

Office européen des brevets

(11) EP 0 182 356 B2

(12) NEW EUROPEAN PATENT SPECIFICATION

(45) Date of publication and mention
of the opposition decision:
05.08.1998 Bulletin 1998/32

(51) Int. Cl.$^6$: **A23L 1/305**, C07K 5/06

(45) Mention of the grant of the patent:
21.08.1991 Bulletin 1991/34

(21) Application number: 85114699.3

(22) Date of filing: 19.11.1985

(54) **Nutrient compositions**

Nährstoffzusammensetzungen

Compositions nutritives

(84) Designated Contracting States:
AT BE CH DE FR GB LI NL SE

(30) Priority: 19.11.1984 US 673010
05.11.1985 US 795193

(43) Date of publication of application:
28.05.1986 Bulletin 1986/22

(73) Proprietors:
• **The Montefiore Hospital Association
of Western Pennsylvania
Pittsburg Pennsylvania 15213 (US)**
• **Pharmacia & Upjohn GmbH
91058 Erlangen (DE)**

(72) Inventors:
• **Adibi, Siamak A.
Pittsburg PA 15217 (US)**
• **Brandl, Maria
D-8540 Schwabach (DE)**
• **Fekl, Werner
D-8551 Rottenbach (DE)**
• **Langer, Klaus
D-8520 Erlangen (DE)**

(74) Representative:
**Freiherr von Pechmann, Eckehart et al
Wuesthoff & Wuesthoff
Patent- und Rechtsanwälte
Schweigerstrasse 2
81541 München (DE)**

(56) References cited:
**EP-A- 0 034 034      EP-A- 0 148 680
EP-B-   87 750        DE-A- 2 260 189
DE-C- 3 108 079       US-A- 4 340 592**

• **Infusionstherapie, 12, 2/1985, pp. 70-76**
• **Gastroenterology, 86, 1984, pp. 1562-1569**
• **J. Nutr,, 108, 1978, pp. 1104-1113**
• **The American Journal of Clinical Nutrition, 29,
1976, pp. 205-215**
• **Clin. Science abd Molecular Medicine, 45, 1973,
pp. 291-299**
• **J. Physiol., 1972, 227, pp. 377-394**
• **Clinical Science, 1972, 43, pp. 659-668**
• **Akt. Ernährung, 4, 1979, pp. 173-177**
• **Defined Formula Diets for Medical Purposes,
1977, pp. 15-24, American Medical Association,
Chicago, III. US**

## Description

Description of the Prior Art - U.S. Patent 4,340,592 and DE-A 3108079 describes nutrient compositions of dipeptides and tripeptides and the method of administering the compositions to mammals for dietary purposes. The important developments described in U.S. Patent 4,340,592 provide a composition which can be employed to supplement nutritional deficiencies or to provide a complete nutritional composition, particularly for a comatose patient or a patient having metabolic or digestive interference. The introduction of large quantities of free amino acids into a mammal tends to establish hypertonicity and metabolic interference.

The expression "protein nutrients" herein includes free amino acids, organic acid amides of amino acids and oligopeptides.

The nutritional problems arising from using free amino acids could be avoided by employing an aqueous mixture containing oligopeptides, that is, dipeptides or tripeptides, of the essential amino acids and other amino acids wherein the N-terminal amino acid is a glycine residue. The glycine terminal amino acid residue achieves water solubility and achieves excellent absorption of the oligopeptides.

While the use of the described aqueous solution of glycine-terminated dipeptides and tripeptides achieves the objectives set forth, there are some improvements which are useful, particularly in providing a complete nutritional composition.

(1) There may be a tendency to develop excess glycine in the patient as a result of using glycine as the N-terminal amino acid residue in all of the oligopeptides. There is no evidence that excess glycine creates any medical problems.

(2) Free amino acids are limitedly soluble in water. Glycine terminated oligopeptides are highly soluble in water. However nutrient compositions containing glycine oligopeptides and/or free amino acids heretofore have been employed in concentrations of 20 weight percent peptide or less, usually less than 15 weight percent total protein content, i.e., the sum of the weight of oligopeptides and the weight of free amino acids. The use of such relatively low concentration aqueous nutrient compositions interferes with development of a complete nutrition system because of the water-intake limits for parenteral nutrition. There is an established limit for the amount of water which can be introduced parenterally into a patient. Approaching that water-intake limit will cause serious problems in medical patients having heart deficiencies or kidney deficiencies. Achieving and exceeding the water-intake limit may be fatal for such patients. Therefore such medical patients cannot be maintained parenterally for extended periods solely by prior art nutrient compositions. Such medical patients heretofore are effectively starving during those periods when they are unable to absorb nutrition except parenterally. It is possible to introduce maintenance-quantities of oligosaccharides, fats, minerals, trace elements and vitamins but it is not possible to introduce sufficient protein ingredients parenterally in the form of free amino acids.

## STATEMENT OF THE PRESENT INVENTION

It is to state that the new aqueous nutrient compositions can be employed in higher concentrations --- up to about 40 weight percent total protein, i.e., the sum of the oligopeptides and the free amino acids --- a much higher range than was heretofore believed to be possible.

The higher concentrations are achieved by combining free amino acids, as desired, with oligopeptides, as desired, to provide the proportions of total protein nutrients which are appropriate to the needs of the patient.

The potential glycine excess can be avoided by providing at least some of the oligopeptides as glycine-terminated oligopeptides and providing other oligopeptides which are terminated with alanine or arginine or lysine moieties. The concentration of the oligopeptides is from 2 to 20 weight percent. The total protein content consisting of free amino acids and oligopeptides is from 10 to 40 weight percent. The high concentrations of protein ingredients permit reduced fluid loads for the patient.

Nutrient compositions having lower concentrations of protein ingredients are useful, particularly with certain essential amino acids such as tryptophan and tyrosine which are difficult to provide as parenteral nutrients.

According to the present invention, novel nutritional compositions are prepared which are aqueous solutions containing at least one oligopeptide consisting of a dipeptide or a tripeptide wherein the N-terminal amino acid residue is glycine residue, <u>and</u>

at least one oligopeptide consisting of a dipeptide or a tripeptide wherein the N-terminal amino acid residue is selected from the class consisting of alanine, lysine and arginine.

The oligopeptide concentration is from 2 to 20 weight percent. For total parenteral nutrition, the preferred range is from 5 to 15 weight percent of the oligopeptide. The total protein nutrients in the compositions are from 10 to 40 weight percent.

The free amino acids are preferably those which can be supplied as aqueous solute and which have stability in storage. The oligopeptides preferably include amino acid moieties which are difficultly soluble in water and those which are unstable in the free amino acid state.

In order to develop a complete nutritional composition, the compositions also may contain other nutrient ingredients such as oligosaccharides, fats, minerals, trace elements, vitamins and free amino acids. The oligopeptides comprise from about 2 to 20 weight percent of the aqueous solution, preferably from 5 to 15 weight percent. The composition also may include free amino acids for the reasons already set forth. The composition is intended for oral, gastrointestinal or intravenous introduction into a patient.

DESCRIPTION OF THE PREFERRED EMBODIMENTS

The composition set forth in Table I has been prepared as a nutrient composition which has been effective to maintain a laboratory animal as a complete nutritional source for an extended period of time.

TABLE I

| Aqueous Nutrient Composition of Investigation 1 | |
|---|---|
| Dipeptide | Concentration (millimoles/liter) |
| Gly-L-Thr | 10.8 |
| Gly-L-Val | 16.3 |
| Gly-L-Met | 7.6 |
| Gly-L-Ile | 12.9 |
| Gly-L-Leu | 18.8 |
| Lys-L-Lys | 5.0 |
| Gly-L-Trp | 3.0 |
| Gly-L-His | 6.0 |
| Gly-L-Phe | 7.6 |
| Arg-L-Glu | 12.6 |
| Gly-L-Pro | 6.0 |
| Gly-L-Ala | 15.0 |
| Gly-L-Tyr | 6.0 |
| Gly-L-Gln | 30.0 |
| Ala-L-Ala | 39.3 |

The conventional abbreviations of Table I are:

| Ala | Alanine | Lys | Lysine |
|---|---|---|---|
| Arg | Arginine | Met | Methionine |
| Gln | Glutamine | Phe | Phenylalanine |
| Glu | Glutamic acid | Pro | Proline |
| Gly | Glycine | Thr | Threonine |
| His | Histidine | Trp | Tryptophan |
| Ile | Isoleucine | Tyr | Tyrosine |

(continued)

| Leu | Leucine | Val | Valine |
|-----|---------|-----|--------|

The Table 1 composition contains all of the essential amino acids and also includes some of the non-essential amino acids. It is well known that the essential amino acids include lysine, leucine, isoleucine, tryptophan, methionine, valine, phenylalanine, threonine. Non-essential amino acids induce arginine, histidine, alanine, proline, glycine, glutamic acid, asparagine, aspartate, cysteine, glutamine, serine, taurine, hydroxyproline, citrulline, alpha-amino-n-butyric acid, cystathionine and ornithine.

It will be observed from Table I that most of the dipeptides in the nutrient composition have a glycine residue in the N-terminal position. However the Lys-L-Lys (Lysyl lysine), the Arg-L-Glu (Arginyl-glutamic acid) and the Ala-L-Ala (Alanyl-alanine) contain lysine, arginine and alanine, respectively as the N-terminal amino acid residue. The composition of Table I has been specially prepared to supply, as peptides, appropriate quantities of the essential amino acids and appropriate quantities of some important non-essential amino acids. By employing some dipeptides which are terminated with the lysine, alanine, arginine residues, the tendency for the patient to develop glycine excess is avoided, in a preferred nutrient composition, the dipeptide formulation of Table I is combined with life-maintenance quantities of oligosaccharides, fats, minerals, vitamins and free amino acids as an aqueous mixture which functions as a complete nutrient composition, particularly for a patient who is comatose or afflicted with gastro-intestinal problems resulting from illness, injury or surgery.

The nutrient compositions of this invention may be administered as described in the aforementioned U.S. Patent 4,340,592, e.g., orally, intragastrointestinally and intravenously.

Additional investigations were carried out with a peptide composition according to the invention set forth in Table 2.

TABLE 2

| Aqueous Nutrient Composition of Investigation 2 --- Protein Nutrient Content 20 Weight Percent | | |
|---|---|---|
| Substance | Concentration | |
| | millimoles per liter | grams per liter |
| glycyl-L-isoleucine | 74.37 | 14.0 |
| L-isoleucine | 38.12 | 5.0 |
| glycyl-L-leucine | 85.00 | 16.0 |
| L-leucine | 41.93 | 5.5 |
| glycyl-L-valine | 137.76 | 24.0 |
| L-valine | 68.29 | 8.0 |
| glycyl-L-tyrosine | 31.46 | 7.5 |
| glycyl-L-glutamine | 44.29 | 9.0 |
| L-alanyl-L-glutamine | 20.72 | 4.5 |
| L-lysine-L-glutamate* | 37.50 | 11.0 |
| L-lysine | 41.04 | 6.0 |
| L-ornithine-L-aspartate* | 28.27 | 7.5 |
| L-arginine | 52.81 | 9.2 |
| L-histidine | 59.29 | 9.2 |
| L-serine | 87.54 | 9.2 |
| L-threonine | 77.23 | 9.2 |
| L-alanine | 207.66 | 18.5 |
| L-proline | 79.91 | 9.2 |
| N-acetyl-L-cysteine* | 3.06 | 0.5 |
| L-methionine | 50.26 | 7.5 |
| L-phenylalanine | 33.29 | 5.5 |
| L-tryptophan | 19.59 | 4.0 |
| Total | | 200.00 |

* - Note
- L-lysine-L-glutamate is a salt of a basic amino acid and an acidic amino acid.
- L-ornithine-L-aspartate is a salt of basic and acidic non-essential amino acids.
- N-acetyl-L-cysteine is an example of an organic acid amide of an amino acid.

It will be observed that the composition of Table 2 includes several free amino acids, one organic acid amide of an amino acid and also includes glycine-terminated dipeptides and includes alanine-terminated dipeptides. The total glycine concentration in this composition was 14 weight percent.

Laboratory animal investigations were carried out with the nutrient composition of Table 2. Effective nutrition was achieved with the laboratory animals over extended periods. The glycine content of the laboratory animal plasma after this investigation was 736. The laboratory animals exhibited no ill effects which might be associated with nutrition.

The urinary excretion of dipeptides during the investigation was less than 1 percent of the amount of infused peptides, which suggests conversion and utilization of the parenterally introduced peptides. The tests established firm evidence for efficacy and safety of a dipeptide mixture as described as the sole nitrogen source for parenteral nutrition in mammals.

A typical nutrient composition for parenteral nutrition having a higher concentration of protein solute than previously contemplated is set forth in Table 4.

TABLE 3

| Aqueous Nutrient Composition of Investigation 3 --- Protein Nutrient Content 40 Weight Percent | | |
|---|---|---|
| Substance | Concentration | |
| | grams per liter | millimoles per liter |
| glycyl-L-isoleucine | 16.1 | 85.54 |
| glycyl-L-leucine | 21.9 | 116.08 |
| glycyl-L-valine | 18.3 | 104.90 |
| L-alanyl-L-tyrosine | 17.6 | 69.76 |
| glycyl-L-glutamine | 35.5 | 174.92 |
| L-glutamic acid | 33.0 | 224.56 |
| L-lysine | 18.0 | 123.06 |
| L-arginine | 37.4 | 214.70 |
| L-histidine | 9.4 | 60.26 |
| L-serine | 37.4 | 355.90 |
| L-threonine | 14.4 | 121.10 |
| L-alanine | 69.5 | 779.66 |
| L-proline | 37.4 | 324.86 |
| N-acetyl-L-cysteine | 0.8 | 4.90 |
| L-methionine | 14.4 | 96.68 |
| L-phenylalanine | 13.4 | 80.86 |
| L-tryptophan | 5.6 | 27.46 |
| Total | 400.0 | |

In Table 4 the nutrient composition includes 11 weight percent peptides including glycine-terminated peptides and one alanyl-terminated peptide (Alanyl-tyrosine). Cysteine is presented as an amide of an organic acid. The total protein content (peptides and free amino acids) is 40 weight percent. Such concentrated, soluble total protein compositions are not proposed in the prior art. The composition of Table 3 contains all of the essential amino acids either as free amino acids or as moieties of an oligopeptide.

**Claims**

1. A parenteral nutrient composition comprising an aqueous solution containing from 10 to 40 weight percent of protein nutrients including 2 to 20 weight percent of oligopeptides consisting of dipeptides and tripeptides wherein at least one said oligopeptide contains a glycine residue as the N-terminal amino acid residue;
    and
    at least one said oligopeptide contains as the N-terminal amino acid residue an amino acid residue selected from the class consisting of alanine, lysine and arginine.

2. The nutrient composition of Claim 1 including alanyl tyrosine as one of the said oligopeptides.

3. The nutrient composition of Claim 1 including arginyl glutamic acid as one of the said oligopeptides.

4. The nutrient composition of Claim 1 including lysyl lysine as one of the said oligopeptides.

5. The nutrient composition of Claim 1 including other nutrients selected from the class consisting of fats, oligosaccharides, minerals, trace elements, vitamins and free amino acids.

6. The nutrient composition of Claim 1 containing all essential amino acids, either as free amino acids or as amino acid residues in the said oligopeptides.

7. The nutrient composition of Claim 1 containing at least one non-essential free amino acid.

8. The nutrient composition of claim 1 comprising dipeptides of L-threonine, L-valine, L-methionine, L-isoleucine, L-leucine, L-lysine, L-tryptophan, L-histidine, L-phenylalanine, L-glutamic acid, L-proline, L-glutamine, L-alanine and L-tyrosine, and, as the N-terminal residue of at least one of said dipeptides, a glycine residue and, as the N-terminal residue of at least one of said dipeptides, at least one other amino acid residue selected from the class consisting of L-alanine, L-lysine and L-arginine.

9. The parenteral nutrient composition of Claim 1 including an organic acid amide of an amino acid.

**Patentansprüche**

1. Parenterale Nährstoffzusammensetzung, umfassend eine wässerige Lösung, die 10 bis 40 Gew.-% Protein-Nährstoffe enthält einschließlich 2 bis 20 Gew.-% Oligopeptide, die aus Dipeptiden und Tripeptiden bestehen, worin mindestens ein Oligtopeptid einen Glycinrest als N-terminalen Aminosäurerest enthält;
und
mindestens ein Oligopeptid als N-terminalen Aminosäurerest einen Alminosäurerest ausgewählt aus der Klasse bestehend aus Alanin, Lysin und Argenin enthält.

2. Nährstoffzusammensetzung nach Anspruch 1, die Alanyltryrosin als eines der Oligopeptide einschließt.

3. Nährstoffzusammensetzung nach Anspruch 1, die Arginylglutaminsäure als eines der Oligopeptide einschließt.

4. Nährstoffzusammensetzung nach Anspruch 1, die Lysyllysin als eines der Oligopeptide einschließt.

5. Nährstoffzusammensetzung nach Anspruch 1, die andere Nährstoffe ausgewählt aus der Klasse, bestehend aus Fetten, Oligosacchariden, Mineralien, Spurenelementen, Vitaminen und freien Aminosäuren einschließt.

6. Nährstoffzusammensetzung nach Anspruch 1, die alle essentiellen Aminosäuren entweder als freie Aminosäuren oder als Aminosäurereste in den Oligopeptiden enthält.

7. Nährstoffzusammensetzung nach Anspruch 1, die mindestens eine nicht-essentielle freie Aminosäure enthält.

8. Nährstoffzusammensetzung nach Anspruch 1, umfassend die Peptide von L-Threonin, L-Valin, L-Methionin, L-Isoleucin, L-Leucin, L-Lysin, L-Tryptophan, L-Histidin, L-Phenylalanin, L-Glutaminsäure, L-Prolin, L-Glutamin, L-Alanin und L-Tyrosin und als N-terminalen Rest mindestens eines der Dipeptide einen Glycinrest, und als N-terminalen Rest mindestens eines der Dipeptide mindestens einen anderen Aminosäurerest ausgewählt aus der Klasse bestehend aus L-Alanin, L-Lysin und L-Arginin.

9. Parenterale Nährstoffzusammensetzung nach Anspruch 1, die ein organisches Säureamid einer Aminosäure einschließt.

**Revendications**

1. Une composition nutritive parentérale comportant une solution acqueuse contenant de 10 à 40 pour-cent en poids d'éléments nutritifs protéiniques y compris 2 à 20 pour-cent en poids d'oligo-peptides se composant de dipeptides et de tripeptides dans lesquels au moins un desdits oligo-peptides contient un résidu de glycine comme le résidu amino-acide N-terminal ;
et
au moins un desdits oligo-peptides contient, en tant que résidu amino-acide en terminaison N, un résidu d'acide aminé sélectionné à partir de la classe se composant d'alanine, de lysine et d'arginine.

2. La composition nutritive de la Revendication 1, incluant de l'alanyl-tyrosine en tant que l'un desdits oligo-peptides.-

3. La composition nutritive de la Revendication 1 incluant de l'arginyl-acide glutamique en tant que l'un desdits oligo-peptides.

4. La composition nutritive de la Revendication 1 incluant de lysyl-lysine en tant que l'un desdits oligo-peptides.

5. La composition nutritive de la Revendication 1 incluant d'autres éléments nutritifs sélectionnés à partir de la classe se composant de graisses, d'oligosaccharides, de minéraux, d'oligoéléments, de vitamines et d'acides aminés libres.

6. La composition nutritive de la Revendication 1 contenant tous les acides aminés essentiels, soit sous la forme d'acides aminés libres soit sous la forme de résidus d'acides aminés dans lesdits oligo-peptides.

7. La composition nutritive de la Revendication 1 contenant au moins un acide aminé libre non essentiel.

8. La composition nutritive de la Revendication 1 comportant les dipeptides de L-thréonine, L-valine, L-méthionine, L-isoleucine, L-leucine, L-lysine, L-tryptophane, L-histidine, L-phénylalanine, L-acide glutamique, L-proline, L-glutamine, L-alanine et L-tyrosine, et, en tant que résidu N-terminal d'au moins l'un desdits dipeptides, un résidu glycine et en tant que résidu en extrémité N d'un au moins desdits dipeptides, au moins un autre résidu d'acide aminé sélectionné à partir de la classe se composant de L-alanine, L-lysine et L-arginine.

9. La composition nutritive parentérale de la Revendication 1 comprenant un amide acide organique d'un acide aminé.